# NEUE EUROPÄISCHE PATENTSCHRIFT

(11) **EP 1 634 946 B2**
(45) Veröffentlichungstag und Bekanntmachung des Hinweises auf die Entscheidung über den Einspruch: **27.07.2011**
(45) Hinweis auf die Patenterteilung: 09.01.2008
(21) Anmeldenummer: 05019746.6
(22) Anmeldetag: 12.09.2005
(51) Int. Cl.: C12M 1/107, B01D 53/047, B01D 53/14, B01D 53/22, C10L 3/10

(54) **Umweltschonendes Verfahren zur Gewinnung von Bioerdgas**
Environmentally safe process for generating biological natural gas
Procédé de production de gaz naturel biologique en respectant l'environnement

(30) Priorität: 13.09.2004 DE 102004044645
(43) Veröffentlichungstag der Anmeldung: 15.03.2006
(73) Patentinhaber: Schmack Carbotech GmbH, 45139 Essen (DE)
(72) Erfinder: Schulte-Schulze Berndt, Alfons Dr., Am Technologiepark 1 45307 Essen (DE); Eichenlaub, Volker, Am Technologiepark 1 45307 Essen (DE)
(74) Vertreter: Minderop, Ralph H.

(56) Entgegenhaltungen:
- WO-A-01/26767
- WO-A-92/12938
- CH-A- 692 653
- DE-A1- 10 047 264
- DE-A1- 10 302 658
- US-A- 1 892 681
- US-A- 4 857 458
- US-A1- 2003 143 719
- US-A1- 2004 103 782
- PATENT ABSTRACTS OF JAPAN Bd. 016, Nr. 020 (C-0902), 20. Januar 1992 (1992-01-20) -& JP 03 236724 A (SUMITOMO SEIKA CHEM CO LTD), 22. Oktober 1991 (1991-10-22) -& DATABASE WPI Section Ch, Week 199148 Derwent Publications Ltd., London, GB; Class C03, AN 1991-351089 XP002360319 & JP 03 236724 A (SUMITOMO SEIKA CHEM CO LTD) 22. Oktober 1991 (1991-10-22)
- H.SEIFERT ET AL.: 'Verfahrenstechnische Lösungen bei der thermischen Abgasreinigung' VDI BERICHTE Nr. 1034, 1993, Seiten 39 - 68
- 'Neue Verbrennungsverfahren für biogene brennstoffe - Verwertung von Reststoffen _ Weniger Schadstoffe' PRESSEMITTEILUNG UNIVERSITÄT STUTTGART 22 April 2004,
- DR. JOACHIM G.WÜNNING: 'FLOX - Flameless Combustion' VDMA 2003 2003,
- [Online] BIO-PRO Gefunden im Internet: <URL:www.eu-projects.de>
- 'New Burner Technologies for Low Grade Biofuels to Supply Clean Energy for Prcesses in Biorefineries' BIO-PRO 17 März 2003,

## Beschreibung

Die Erfindung betrifft ein Verfahren zur Gewinnung eines Bioerdgases aus Methan enthaltenden Rohbiogasen in einer Biogasaufbereitungsstufe.

Unter Bioerdgas wird ein mit Methan angereichertes Gas verstanden, das durch geeignete Verfahren aus Rohbiogasen gewonnen wird und einen erdgasgleichen oder höherwertigen Charakter aufweist.

Rohbiogase sind im allgemeinen Fermentationsgase aus der anaeroben Fermentation organischer Biomasse. Organische Biomasse schließt beispielsweise Haushalts- und Industrieabfälle, pflanzliche und tierische Rückstände, organische Rückstände von Kläranlagen und nachwachsende Rohstoffe ein. Charakteristischer Bestandteil für durch Fermentation gewonnene Biogase ist das aufgrund des biochemischen Abbaus von organischen Massen gebildete Methan (CH₄). Ein weiterer wesentlicher Bestandteil neben Methan ist Kohlendioxid (CO₂).

Zur Erzeugung von Rohbiogas wird die Biomasse zunächst konditioniert und als Gärsubstrat in den Fermentationsbehälter gegeben. Die Konditionierung schließt Verfahrensmaßnahmen wie Zerkleinern, Sieben, Sortieren und mit Wasser verdünnen ein. Im Fermentationsbehälter erfolgt bei Temperaturen von 30 bis 40°C unter Luftausschluss die bakterielle Zersetzung der Biomasse unter Biogasbildung. Das erhaltene Biogas wird vorgereinigt und über Gaspuffer der weiteren Verwendung zugeleitet. Ein Teil des Biogases, beispielsweise 10 bis 20 Vol.% in Abhängigkeit von der Anlagengröße, der Prozesstechnik und der Jahreszeit, dient der Erzeugung von Wärme und Strom direkt vor Ort. Dadurch wird der Eigenwärmebedarf der Biogaserzeugungsanlage gedeckt. Die Wärme- und Stromerzeugung erfolgt in der Regel durch sogenannten Blockheizkraftwerke. Da die Blockheizkraftwerke primär der Wärmeerzeugung dienen, arbeiten sie wärmegeführt, der elektrische Wirkungsgrad ist deshalb eher gering.

Rohbiogase können neben den beiden Hauptbestandteilen Methan (CH₄) und Kohlendioxid (CO₂) eine Vielzahl weiterer Bestandteile, wenn auch teilweise nur im Spurenbereich, enthalten. Die Zusammensetzung des Rohbiogases variiert in einem weiten Bereich. Die folgende Tabelle 1 gibt Anhaltswerte.

**Tabelle 1 - Gaszusammensetzung der Biogase**

| | |
|---|---|
| CH₄ | 40 - 70 Vol.% |
| CO₂ | 30 - 60 Vol.% |
| N₂ | 0 - 7 Vol.% |
| O₂ | 0 - 3 Vol.% |
| H₂S | 0 - 3.000 mg/Nm³ |
| Mercaptane | 0 - 100 mg/Nm³ |
| BTX | 0 - 500 vpm |
| Höhere Kohlenwasserstoffe | 0 - 500 vpm |
| Halogenierte Kohlenwasserstoffe | 0 - 500 vpm |
| Ammoniak | 0 - 100 mg/Nm³ |
| Wasser | Gesättigt |
| Brennwert | 4 - 8 kwh/Nm³ |

Zur Gewinnung eines Biogases mit Erdgasqualität (Bioerdgas) aus Rohbiogas müssen die Hauptbestandteile Kohlendioxid und die übrigen Verunreinigungen weitestgehend entfernt werden. Die Anforderungen an die Qualität von Bioerdgas für das Einleiten oder Durchleiten in oder durch Erdgastransportleitungen oder die Verwendung als Austauschgas für Erdgas werden durch Regelwerke mit Verordnungscharakter festgelegt. Zu nennen sind hier insbesondere die DVGW G 260, 261 und 262; deren wesentliche Anforderungen sind in Tabelle 2 wiedergegeben.

**Tabelle 2 - Gaszusammensetzung mit Erdgasqualität gemäß DVGW**

| | |
|---|---|
| Methan | >96 |
| Kohlendioxid | Keine Mindestwerte |
| Stickstoff | Keine Mindestwerte |
| Sauerstoff | < 0,5 Vol.% |
| Schwefelwasserstoff | < 5 mg/Nm3 |
| Kohlenwasserstoffe | < Kondensationspunkt |
| Wasser | < Kondensationspunkt |

Ein Bioerdgas, das die oben genannten Anforderungen erfüllt, wird in der Regel durch Einsatz mehrstufiger Verfahren erzielt. Dabei werden durch Filtration, Kondensation, Absorption, Adsorption und/oder Chemiesorption Verunreinigungen wie Schwefelwasserstoff (H₂S), andere Schwefelverbindungen, BTX, höhere Kohlenwasserstoffe und vor allem Kohlendioxid entfernt oder teilentfernt. Je nach Zusammensetzung des Rohbiogases ist ein mehr oder weniger aufwendiges Vorreinigen zum Entfernen von Schwefelwasserstoff, Ammoniak und höheren Kohlenwasserstoffen erforderlich.

Allen bekannten Verfahren zur Rohbiogasaufbereitung oder zur Methan-Anreicherung ist gemeinsam, dass trotz höchster Effizienz die Ausbeute an Methan teilweise deutlich unter 100 Vol.% liegt; meist liegen sie im Bereich von 95 bis 99 Vol.%. Dies führt zwangsläufig zu hohen Methanverlusten.

Das Abgas wird üblicherweise ohne weitere Behandlung direkt in die Atmosphäre abgegeben. Darüber hinaus enthält das Abgas noch Verunreinigungen des Rohbiogases, deren Konzentration in Abhängigkeit von dem gewählten Aufbereitungsverfahren jedoch stark unterschiedlich ist.

Kohlendioxid ist bekannt als verantwortliches Gas für den sogenannten Treibhaus-Effekt. Methan ist aber als Treibhausgas um den Faktor 21 schädlicher als Kohlendioxid. Aus diesem Grunde sollte, soweit technisch vertretbar, Methan nicht in die Atmosphäre abgegeben werden. Darüber hinaus ist zu bedenken, dass durch die bekannte Verfahrensweise ein erheblicher Anteil an Methan im Abgas als nutzbarer Brennstoff verloren geht.

Die JP 03-236724 schlägt vor, Rohbiogas einer Behandlung zur Trennung von Kohlendioxid und Methan zu unterziehen. Das gewonnene Methan enthaltende Gas wird zur Erwärmung eines Treibhauses oder zur Gewinnung von thermischer Energie in einer wärmeerzeugenden Maschine eingesetzt. Das an kohlendioxidreiche Gas wird den Pflanzen zur Photosynthese zur Verfügung gestellt. Die WO 92/12938 offenbart ein Verfahren zur Erzeugung von Rohbiogas und dessen Aufbereitung durch Trennung des Rohbiogases in ein relativ kohlendioxidreiches Gas und ein an Kohlendioxid relativ armes Gas. Das kohlendioxidreiche Gas soll den Pflanzen eines Treibhauses zur Photosynthese zur Verfügung gestellt werden, das Gas mit einem hohen Methangehalt soll als Brennstoffquelle genutzt werden. Die DE 100 47 264 A1 offenbart ein Verfahren zur Nutzung von methanhaltigem Biogas, wobei Kohlendioxid abgetrennt wird, das methanreiche Gas verbrannt werden soll und das kohlendioxidreiche Gas in den Deponiekörper oder Faulturm rückgeführt werden soll.

Die US 4 857 458 beschreibt ein Verfahren zur Gewinnung von Rohbiogas. Bevor das Fermentations-produkt gelagert wird, wird es einer Reinigung unterzogen.

Der Erfindung liegt daher die Aufgabe zugrunde, ein Verfahren zur Aufbereitung von Rohbiogas zu Biogas mit Erdgasqualität bereitzustellen, in dem die Methanabgabe in die Atmosphäre vermieden wird und das dennoch eine energieeffiziente Verfahrensweise ermöglicht.

Gelöst wird diese Aufgabe durch ein Verfahren zur Gewinnung eines Bioerdgases aus Methan enthaltenden Rohbiogasen in einer Biogasaufbereitungsstufe, wobei man bei der Aufbereitung anfallendes, Methan enthaltendes Abgas thermisch in der Erzeugung des Rohbiogases nutzt und kein Methan enthaltendes Gas in der Atmosphäre freisetzt und in dem die Biogasaufbereitungsanlage nach dem Prinzip des Druckwechseladsorptionsverfahrens oder nach dem Prinzip der Druckwasserwäsche oder nach dem Prinzip der Permeationstrennung mit Membran oder nach dem Prinzip der Wäsche mit Kohlendioxid-Adsorptionsmittel betrieben wird.

Die Aufbereitung des Rohbiogases kann also derart durchführt werden, dass das Abgas der Aufbereitungsstufe einen Methangehalt aufweist, dessen Brennwert den Eigenwärmebe darf der Rohbiogaserzeugung deckt. Das Abgas kann zur Erwärmung von Wasser verbrannt werden. Die im Wasser gespeicherte Wärmeenergie wird der Rohbiogaserzeugung (Fermentation) zugeführt.

Gemäß einer besonders bevorzugten Ausführungsform der Erfindung weist das Abgas etwa den Brennwert auf, der notwendig ist, um den Eigenwärmebedarf der Biogaserzeugung unter Berücksichtigung der Kesselverluste und sonstigen Verluste zu decken. Das Abgas kann beispielsweise eine Konzentration an Methan von etwa 10 Vol.%, vorzugsweise 14 Vol.% und besonders bevorzugt etwa 15 Vol.% und mehr aufweisen.

Das Abgas wird mithin nicht wie bei den bekannten Verfahren üblich in die Atmosphäre abgegeben, sondern beispielsweise einem Warmwasserkessel mit speziellem Schwachgasbrenner zur Verbrennung und Wärmeerzeugung zugeleitet.

Dazu wird erfindungsgemäß die Biogasaufbereitung bewußt mit einem schlechteren Wirkungsgrad als bei bekannten Verfahren gefahren, d.h. die Ausbeute an Methan im Biogas mit Erdgasqualität ist geringer als bei den bekannten Verfahren.

Die Einstellung eines "schlechteren Wirkungsgrads" bedingt nun deutlich höhere Konzentrationen an Methan im Abgas, verglichen mit den bekannten Verfahren, und damit zunächst theoretisch höhere Verluste. Durch die komplette thermische Nutzung des Abgases zur Erzeugung von Nutzwärme, vorzugsweise warmem Wasser, wird aber nicht nur das bisher an die Atmosphäre abgegebene Methan energetisch sinnvoll genutzt, sondern auch die weiteren im Abgas befindlichen Verunreinigungen genutzt oder thermisch unschädlich gemacht. Methan wird vollständig zu Kohlendioxid und Wasser und geringen Spuren an Kohlenmonoxid und NOₓ umgesetzt und kann über den Kesselabgasstrom der Atmosphäre zugeführt werden. Da das Kohlendioxid dem natürlichen Kohlendioxid-Kreislauf zugefügt wird, ist auch dieses als umweltneutral zu beurteilen.

Eine weitere positive Wirkung gegenüber dem herkömmlichen Verfahren ist die nun ebenfalls höhere Bioerdgasproduktion bei gleichem Rohbiogaseinsatz und die höhere gesamte energetische Nutzung. Eine direkte Nutzung des Abgases der bekannten Aufbereitungsverfahren ist entweder nicht möglich oder führt zu weiteren Nachteilen. Würde nämlich das Abgas der Biogasaufbereitung nach den bekannten Verfahren mit dem Betriebsgas (Rohbiogas) eines Blockheizkraftwerks unter Beibehaltung des gleichen notwendigen Energieinputs vermischt, so erhielte man ein Gas mit einem Anteil an Methan von deutlich unter 40 Vol.%. Mit solchen sogenannten Schwachgasen lassen sich Blockheizkraftwerke nicht mehr betreiben. Darüber hinaus ist eine Verbrennung des Schwachgases mit dem geringen Anteil an Methan in Abhitzkesseln mit Schwachgasbrenner ohne zusätzliche Stützfeuerung nicht möglich.

Eine zumindest teilweise Rezirkulation von Abgas aus der Rohbiogasaufbereitung ist nicht ausgeschlossen. Letztendlich wird das Abgas aber zur Erzeugung von Nutzwärme verwendet. Anders als in den bekannten Verfahren wird kein Methan in die Atmosphäre freigesetzt.

Als Rohbiogas können alle üblichen Methan enthaltenden Biogase aus Fermentationsanlagen (Biorektoren) eingesetzt werden. Die Rohbiogase können beispielsweise erzeugt werden aus Stoffen aus der landwirtschaftlichen Produktion, Klärschlämmen, Abfällen aus bioindustriellen Prozessen und aus anderen organischen Abfällen. Die erfindungsgemäß als Ausgangsmaterial eingesetzten Rohbiogase können eine Konzentration an Methan von 50 bis 70 Vol.%, vorzugsweise mindestens etwa 60 Vol.%, aufweisen.

In vorgeschalteten Verfahren können einzelne Verunreinigungen wie Schwefelwasserstoff entfernt werden. Diese Verfahren sind bekannt.

Die erfindungsgemäße Gewinnung von Bioerdgas wird ohne Verschaltung mit einem Blockheizkraftwerk durchgeführt. Das bedeutet, dass kein Teilstrom des Rohbiogases in ein Blockheizkraftwerk zur Verbrennung geleitet wird.

Zur Aufbereitung des Rohbiogases mit dem Ziel, ein Biogas mit Erdgasqualität zu erhalten, werden die bekannten Verfahren Druckwasserwäsche, das Druckwechseladsorptionsverfahren, die Permeationstrennung mit Membran oder die Wäsche mit speziellen CO₂-Absorptions mitteln wie Aminen eingesetzt. Diese Verfahren sind grundsätzlich bekannt und sind zu dem genannten Zweck beschrieben worden.

Bei der sogenannten Druckwasserwäsche wird verdichtetes Rohbiogas in eine Waschkolonne im Gegenstrom zu Wasser eingebracht. Wasser absorbiert das im Rohbiogas enthaltene CO₂. In diesem Verfahren wird die unterschiedliche Löslichkeit von Kohlendioxid und Methan in Wasser genutzt. Kohlendioxid löst sich unter Druck stehend stärker als Methan im Wasser und wird bei einem Rückgang des Behälterdrucks wieder freigesetzt. Bei Betriebsdrücken von 5 bis 15 bar mit einer Absorptionstemperatur von etwa 20°C wird CO₂ in einer Gegenstrom-Packungskolonne mittels Brauchwasser absorbiert. Das mit Methan angereicherte Biogas wird am Kopf der Kolonne abgenommen. Das mit Kohlendioxid angereicherte Wasser kann in einer der Waschkolonnen nachgeschalteten Kolonne auf Atmosphärendruck entspannt werden. Dadurch wird Kohlendioxid freigesetzt. Das Gemisch aus Wasser und Kohlendioxid kann in einen Stripper überführt werden, in den Luft eingeblasen und damit ein Luft/CO₂-Gemisch ausgetragen wird. Das Wasser kann wieder in die Waschkolonne rückgeführt werden.

Ein_solches Druckwasserwäsche-Verfahren mit einer physikalischen Wäsche ist beispielsweise beschreiben in Weiland, Peter: Notwendigkeit der Biogasaufbereitung, Ansprüche einzelner Nutzungsruten und Stand der Technik, Vortrag im Rahmen des FNR-Workshops "Aufbereitung von Biogas" am 17.06.2003 in Braunschweig.

Ein weiteres geeignetes Verfahren zur Aufbereitung von Rohbiogas ist die Wäsche mittels spezieller Absorptionsmittel für Kohlendioxid. Man spricht hier auch von einer chemisch-physikalischen Wäsche, bei der die Waschlösung und die zugehörige chemische Reaktion, beispielsweise mit Monoethanolamin, von Bedeutung ist. Bei diesem Verfahren findet eine geringere Verdichtung des Rohbiogases statt als in dem Druckwasserwäscheverfahren.

Ein weiteres Aufbereitungsverfahren für Rohbiogas ist das sogenannte Druckwechseladsorptionsverfahren. Dieses Verfahren ist ein adsorptives oder sogenanntes "trockenes Verfahren" der Kohlendioxidabtrennung. Das Biogas wird über einen Verdichter in die Adsorberbehälter gepresst. Der eingesetzte Druck kann beispielsweise bei etwa 0,7 x 10⁶ Pa (17 bar) liegen. Dort adsorbiert das Kohlendioxid an Aktivkohle oder an Molekularsiebe auf Kohlenstoffbasis. Um die Gasreinigung während der Regeneration eines Filters nicht unterbrechen zu müssen und den Energiebedarf für die Gaskompression zu reduzieren, indem der freigesetzte Gasdruck jedes Adsorbers in den anderen genutzt werden kann, werden mindestens zwei, vorzugsweise vier Adsorberbehälter zusammengeschlossen. Das am Ende erhaltene methanreiche Gas kann in die Gasleitung eingespeist werden. Das bei der Druckentspannung desorbierte und unter Anlegen eines Vakuums abgesaugte CO₂ wird in die Atmosphäre abgeführt. Auch dieses Verfahren ist beispielsweise beschrieben in dem zuvor zitierten Vortrag von Peter Weiland.

Schließlich kann die Anreicherung des Methans im Rohbiogas durch eine Permeationstrennung mit einer Membran erfolgen. Das sogenannte Membran-Trennverfahren nutzt die unterschiedlichen Permeabilitäten des Membranmaterials für die verschiedenen Gasmoleküle. Der eingesetzte Werkstoff entscheidet über die Selektivität. Mit der Membrantrennung kann daher sowohl die gemeinsame Abtrennung von Kohlendioxid und Schwefeldioxid als auch die selektive Abtrennung von H₂S und CO₂, beispielsweise in zweistufigen Anlagen, durchgeführt werden. Die abgetrennten Gasbestandteile werden in einem nächsten Schritt in Waschlösungen absorbiert (nasses Membranverfahren) oder gasförmig ausgetragen (trockenes Membranverfahren). Das für den Trennprozess erforderliche Druckgefälle wird in der Regel durch einen Überdruck auf der Rohgasseite realisiert. Das aufbereitete Gas liegt bei einem vergleichsweise hohen Druck vor, der eine Einspeisung in das öffentliche Gasnetz erleichtert. Die Membranen sind häufig solche, die Hohlfasern enthalten. Dieses Verfahren ist beschrieben in Schulte-Schulze Berndt, Alfons: Gasaufbereitung mittels Druckwechseladsorption, Vortrag im Rahmen des FNR-Workshops "Aufbereitung von Biogas" am 17.06.2003 in Braunschweig.

Allen bekannten Verfahren ist gemeinsam, dass Methan über das Abgas in die Atmosphäre gelangt.

Bei der Aufbereitung von Rohbiogas nach den bekannten Verfahren wird ein Teilstrom des Abgases vollständig oder teilweise rezirkuliert, um Ausbeuten an Methan von 95 bis 99 Vol.% zu erreichen. Eine solche Rezirkulation des Abgases in der Rohbiogas-Aufbereitungsanlage findet im erfindungsgemäßen Verfahren vorzugsweise nicht statt.

Zur Einstellung einer höheren Methanmenge im Abgas der Rohbiogasaufbereitung nach dem Verfahren der Druckwasserwäsche kann der Druck, die Wassermenge und/oder die zum Strippen eingesetzte Luft variiert werden.

Wird bei der Druckwasserwäsche eine geringere Verdichtung des Rohbiogases vorgenommen, erhöht sich die Konzentration von Methan im Abgas. Dasselbe gilt für die Verringerung der Menge an Waschwasser. Eine höhere Methankonzentration im Abgas kann auch dadurch erreicht werden, dass die zum Strippen eingesetzte Menge an Luft verringert wird.

Bei der chemisch-physikalischen Wäsche mittels spezieller Kohlendioxid-Absorptionsmittel kann die Erhöhung des Anteils an Methan im Abgas bewirkt werden durch eine geringere Menge des Absorptionsmittels oder durch Verringerung des Drucks.

Im Druckwechseladsorptionsverfahren kann eine höhere Konzentration von Methan im Abgas durch kürzere Zykluszeiten der Filter bewirkt werden. Je häufiger auf einen anderen Filter umgeschaltet wird, desto mehr Gasverluste entstehen. Dies führt zu einer Anreicherung von Methan im Abgas.

Eine höhere Konzentration bei der Aufbereitung von Rohbiogas in einer Permeationstrennung mit Membran kann durch Verringerung der Anzahl der Trennstufen erfolgen, beispielsweise durch eine nur zweistufige Aufbereitung.

Erfindungsgemäß besonders bevorzugt wird die höhere Methanmenge im Abgas durch Weglassen der bekannten Rezirkulation des Abgases der Aufbereitung in die Aufbereitungsstufe. Das gilt für alle Aufbereitungsverfahren.

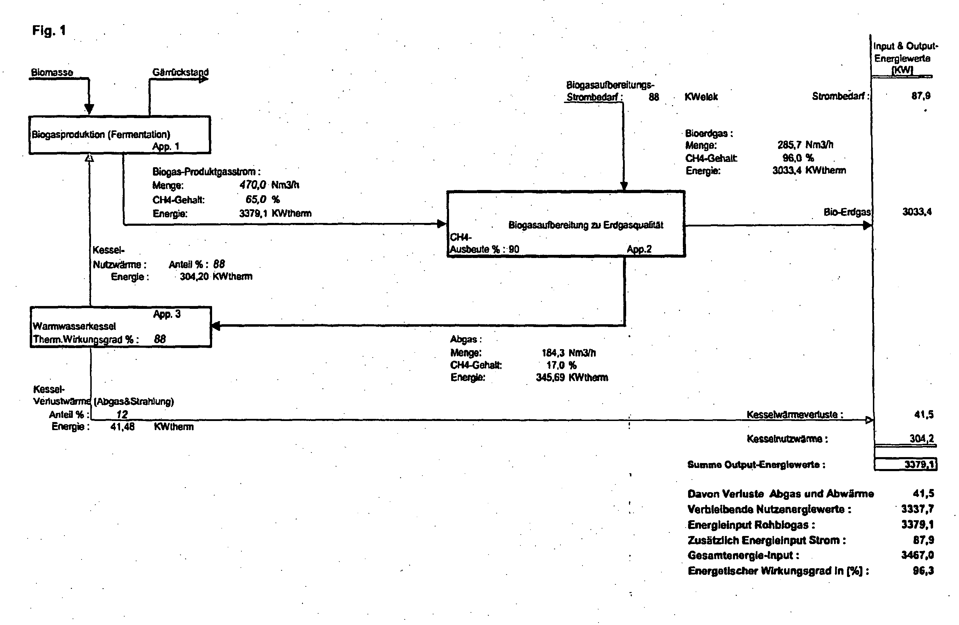

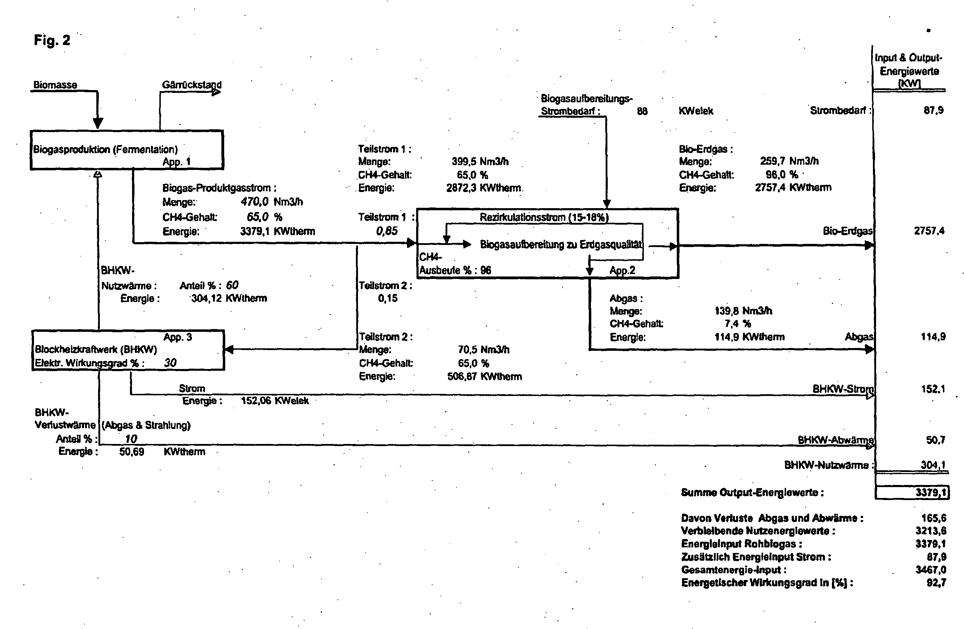

Fig. 1 zeigt eine Aufbereitung von Rohgas nach dem erfindungsgemäßen Verfahren. Fig. 2 zeigt die bekannte Aufbereitung von Rohbiogas.

Das erfindungsgemäße Verfahren wird nachfolgend beispielhaft unter Bezugnahme auf Fig. 1 erläutert. Aus der Biogaserzeugung werden 470,0 Nm³/h Rohbiogas mit einem Methangehalt von etwa 65 Vol.% in die Aufbereitungsstufe eingeleitet. Die thermische Energie des Rohbiogases beträgt 3379,1 KW. Die Biogasaufbereitung erfolgt nach dem Druckwechselabsorptionsverfahren. Dazu wird das Rohbiogas auf etwa 6 x 10⁵ Pa (6 Bar) verdichtet, Wasser abgeführt und der verdichtete Rohbiogasstrom bei etwa 20 °C in die Adsorberbehälter gepreßt. Die Adsorberbehälter sind mit einem Molekularsieb auf Kohlenstoffbasis befüllt. Das mit Methan angereicherte Gas wird in die Gasleitung eingespeist. Das bei der Druckentspannung desorbierte Kohlenstoffdioxid und andere gasförmige Verunreinigungen werden unter Anlegen eines Vakuum abgesaugt und in die Atmosphäre abgeführt. Bei dieser Verfahrensweise erfolgt keine Rezirkulation des in der Aufbereitungsstufe anfallenden Abgases. Bei dieser Verfahrensführung der Biogasaufbereitung ohne Rezirkulation wird eine Methanausbeute von 90 Vol.% erzielt. Der Strombedarf für die Biogasaufbereitung beträgt 88 KWelek.

184,3 Nm³/h Abgas mit einem Gehalt an Methan von 17 Vol.% und 345,69 KWtherm werden aus der Biogasaufbereitungsstufe abgeführt und verbrannt. Die Wärmeenergie dient der Erwärmung von Wasser in einem Warmwasserkessel. Die Verbrennung erfolgt mit einem flammlosen Schwachgasbrenner ohne Stützgasfeuerung. Der thermische Wirkungsgrad der Wassererwärmung beträgt 88 Vol.%, d.h. 304,20 KWtherm gehen als Kesselnutzwärme in die Fermentation. Die Kesselverlustwärme macht mithin einen Anteil von 12 Vol.% (41,48 KWtherm).

Die Kesselnutzwärme, im vorliegenden Fall 304,2 KW, wird in die Biogasproduktion überführt und dort beispielsweise zum Aufrechterhalten der Fermentationstemperatur zwischen 30 und 40°C verwendet.

Erfindungsgemäß werden 285,7 Nm³/h Bioerdgas mit einer Methankonzentration von 96 Vol.% und einem Energiegehalt von 3033,4 KWtherm erzielt. Die Methankonzentration im Bioerdgas ist mithin dieselbe wie in den bekannten Verfahren. Das angereicherte Biogas weist Erdgasqualität auf. Der gesamte energetische Wirkungsgrad liegt somit bei 96,3 %.

Die Vorteile des erfindungsgemäßen Verfahrens bestehen darin, dass Methan-Emissionen vermieden werden, eine etwa 10 % höhere Bioerdgasproduktion bei gleicher Rohbiogasmenge wie im herkömmlichen Verfahren erzielt wird, der gesamte energetische Wirkungsgrad deutlich höher ist, deutlich geringere Investitionen erforderlich sind, da nur ein Warmwasserkessel und nicht ein Blockheizkraftwerk zusätzlich erforderlich ist. Die Investitionskosten für den Warmwasserkessel betragen daher nur etwa ein Drittel der üblichen Kosten eines Blockheizkraftwerkes. Die Prozeßführung ist bei dem erfindungsgemäßen Aufbereitungsverfahren viel einfacher, da keine Rezirkulation des angereicherten Gases zur Erhöhung der Ausbeute an Methan erforderlich ist. Die Bedienung und Wartung der erfindungsgemäß erforderlichen Anlage ist weniger intensiv als wenn zusätzlich ein Blockheizkraftwerk vorhanden ist. Dadurch sinken auch die Wartungskosten.

Zum Vergleich mit einem erfindungsgemäßen Verfahren wird nachfolgend die Durchführung des bekannten Aufbereitungsverfahrens unter Bezugnahme auf Fig.2 erläutert.

Aus 470 Nm³/h Rohbiogas aus der Fermentation mit einem Methangehalt von 65 Vol.% und damit einem Energiegehalt von 3379,1 KWtherm wird ein Teilstrom abgeleitet. Teilstrom 1 enthält 399,5 Nm³/h Rohbiogas mit einem Energiegehalt von 2872,3 KWtherm. Teilstrom 2 enthält 70,5 Nm³/h Rohbiogas und damit eine Energie von 506,87 KWtherm. Der Eigenwärmebedarf der Fermentation von 304 KWtherm wird durch die Verwendung des Teilstroms 2 (15 vol.% der Rohbiogasmenge) in einem Blockheizkraftwerk (BHKW) gedeckt. Als weiteres energetisch nutzbares Nebenprodukt fällt Strom bei einem elektrischen Wirkungsgrad von 30 Vol.% an.

Der verbleibende größere Teilstrom 1 (85 Vol.% der Rohbiogasmenge) gelangt zur Biogasaufbereitung. Diese erfolgt nach dem Druckwechselabsorptionsverfahren wie zuvor für das erfindungsgemäße Verfahren beschrieben. Durch die verfahrenstechnische Optimierung, im vorliegenden Fall durch Rezirkulation eines Teils des Methan enthaltenden Abgases, wird eine hohe Methanausbeute von 96 Vol.% erzielt. Der Eigenstrombedarf (Verdichter, Pumpen) der Biogasaufbereitung liegt bei 88 KWelekt.

Die erhaltene Bioerdgasmenge beträgt etwa 260 Nm³/h mit einem Energieinhalt von 2757,4 KWtherm, die Abgasmenge beträgt etwa 140 Nm³/h mit einem Gehalt an Methan von 7,4 Vol.% und einem Energieinhalt von 114,9 KWtherm. Der gesamte energetische Wirkungsgrad liegt somit bei 92,7 %.

In Tabelle 3 werden im Hinblick auf die Energie wesentlichen Daten der beiden Verfahren gegenübergestellt.

**Tabelle 3 - Vergleich der Verfahren gemäß Fig. 1 und 2**

| | Erfindungsgemäßes Verfahren | Stand der Technik |
|---|---|---|
| Verluste (Abgas, Abwärme) | 41,5 KW | 165,6 KW |
| Verbleibende Nutzenergie | 3337,7 KW | 3213,6 KW |
| Energieinput Rohbiogas | 3379,1 KW | 3379,1 KW |
| Zusätzlicher Energieinput Strom | 87,9 KW | 87,9 KW |
| Gesamtenergieinput | 3467,0 KW | 3467,0 KW |
| Energetischer Wirkungsgrad in [%] | 96,3 | 92,7 |

Bedingt durch Änderungen und Wechsel der Biomasse sowie Störungen/Änderungen der Biologie im Fermenter bei der Robhiogaserzeugung kann der Methangehalt und/oder die Rohbiogasproduktion teilweise deutlich variieren. Um die damit verbundenen unterschiedlich notwendigen Wärmemengen schnell und einfach abdecken zu können, wird in einer besonderen Ausführungsform des erfindungsgemäßen Verfahrens das Abgas der Biogasaufbereitung mit einem Rohbiogas, einem teilaufbereitetem Rohbiogas und/oder Bioerdgas gemischt und dem Warmwasserkessel zur Verbrennung zwecks Wärmeerzeugung zuführt.

Dazu kann ein Teilstrom des Rohbiogases, eines teilaufbereiteten Rohbiogases oder des erzeugten Biogases mit dem Abgas gemischt und das Mischgas thermisch genutzt werden, insbesondere dem Warmwasserkessel zwecks Wärmeerzeugung für die Biogasproduktion zugeleitet werden.

Beispielsweise wird wie im Ausführungsbeispiel gemäß Fig. 1 ein Rohbiogasstrom von 470 Nm³/h produziert, der jedoch, beispielsweise wegen eines geringeren Fettanteils in der Biomasse, nur 55 Vol.% Methan enthält; der Wärmebedarf der Fermentation in Höhe von etwa 304 KW ist gleich wie im Beispiel nach Fig.1. Ein Teilstrom, 1,459 Nm³/h, gelangt zur Biogasaufbereitung nach dem Druckwechseladsorptionsverfahren. Das Abgas, etwa 222 Nm³/h mit 11,4 Vol.% Methan, wird mit einer kleinen Menge Rohbiogas als Teilstrom (11 Nm³/h) gemischt und dem Warmwasserkessel zwecks Verbrennung und Wärmeerzeugung zugeführt. Die Teilstrommenge wird in der Praxis automatisch so geregelt, dass der jeweils aktuelle Wärmebedarf der Biogasproduktion aus dem Abgas sowie dem Teilstrom Rohbiogas gedeckt werden kann. Bei dieser Verfahrensweise beträgt der Energieinput Rohbiogas 2859,3 KW. Es ergeben sich Verluste für Abgas und Abwärme von 41,5 KW. Daraus ergibt sich ein verbleibender Nutzenergiewert von 2817,17 KW. Bei einem zusätzlichen Energieinput (Strom) von 85,8 KW ist der Gesamtenergieinput 2945,1. Der energetische Wirkungsgrad beträgt mithin 95,7 % und liegt über dem des bekannten Verfahrens nach Fig. 2.

## Patentansprüche

1. Verfahren zur Gewinnung eines Bioerdgases aus Methan enthaltenden Rohbiogasen in einer Biogasaufbereitungsstufe, **dadurch gekennzeichnet, dass** man bei der Aufbereitung des Rohbiogases anfallendes, Methan enthaltendes Abgas thermisch in der Erzeugung des Rohbiogases nutzt und kein Methan enthaltendes Gas in die Atmosphäre freisetzt und die Biogasaufbereitungsanlage nach dem Prinzip des Druckwechseladsorptionsverfahrens oder nach dem Prinzip der Druckwasserwäsche oder nach dem Prinzip der Permeationstrennung mit Membran oder nach dem Prinzip der Wäsche mit Kohlendioxid-Absorptionsmitteln betrieben wird.

2. Verfahren nach Anspruch 1, **dadurch gekennzeichnet, dass** man Abgas einem Warmwasserkessel zwecks Verbrennung und Wärmeerzeugung zuführt.

3. Verfahren nach einem der Ansprüche 1 oder 2, **dadurch gekennzeichnet, dass** man einen Warmwasserkessel zur Deckung des Eigenwärmebedarfs der Biogaserzeugungsanlage mit einem flammlosen Schwachgasbrenner ohne Stützgasfeuerung betreibt.

4. Verfahren nach einem der Ansprüche 1 bis 3, **dadurch gekennzeichnet, dass** man im Abgas der Biogasaufbereitungsanlage ein energetisch höherwertiges Abgas erzeugt, indem man auf die Rezirkulation des Abgases der Anlage verzichtet.

5. Verfahren nach einem der Ansprüche 1 bis 4, **dadurch gekennzeichnet, dass** man das Abgas der Biogasaufbereitungsanlage mit Rohbiogas, teilaufbereitetem Rohbiogas und/oder Bioerdgas mischt und dem Warmwasserkessel zur Verbrennung zwecks Wärmeerzeugung zuführt, um den wechselnden Energiebedarf der Biogasproduktion abzudecken.

## Claims

1. A method for obtaining a natural biogas from crude biogases containing methane in a biogas processing stage, **characterised in that** exhaust gas produced during processing of the crude biogas is used thermally in the production of crude biogas and no gas containing methane is released into the atmosphere and the biogas processing plant is operated on the principle of the pressure change adsorption process or on the principle of pressure water washing or on the principle of permeation separation with membranes or on the principle of washing with carbon dioxide absorption means.

2. The method according to Claim 1, **characterised in that** exhaust gas is supplied to a hot water boiler for the purpose of combustion and heat production.

3. The method according to Claims 1 or 2, **characterised in that** a hot water boiler for covering the internal heat requirement of the biogas production plant is operated with a flameless poor gas burner without auxiliary gas firing.

4. The method according to one of Claims 1 to 3, **characterised in that** a high energy exhaust gas is produced in the exhaust gas of the biogas processing plant by dispensing with recirculation of the exhaust gas of the plant.

5. The method according to one of Claims 1 to 4, **characterised in that** the exhaust gas of the biogas processing plant is mixed with crude biogas, partially processed crude biogas and/or natural biogas and is supplied to the hot water boiler for combustion for the purpose of heat generation in order to cover the changing energy requirement for biogas production.

## Revendications

1. Procédé d'extraction d'un biogaz naturel à partir de biogaz brut contenant du méthane au cours d'une étape de traitement de biogaz, **caractérisé en ce que** l'on utilise un gaz résiduaire contenant du méthane, résultant du traitement du biogaz brut, thermiquement dans la production du biogaz brut, et que l'on ne libère pas de gaz contenant du méthane dans l'atmosphère et l'étape de traitement de biogaz est exploiter selon le principe du procédé d'adsorption par variation de la pression ou selon le principe du lavage à l'eau sous pression ou selon le principe de la séparation par perméation à l'aide de membranes ou selon le principe du lavage à l'aide d'agents d'absorption de l'anhydride carbonique.

2. Procédé selon la revendication 1, **caractérisé en ce que** l'on conduit le gaz résiduaire dans une chaudière à eau chaude en vue d'une combustion et d'une production de chaleur.

3. Procédé selon l'une des revendications 1 ou 2, **caractérisé en ce que** l'on exploite une chaudière à eau chaude pour couvrir les besoins propres en chaleur de l'installation de traitement du biogaz, avec un brûleur à gaz pauvre sans flamme, sans combustion de gaz auxiliaire.

4. Procédé selon l'une des revendications 1 à 3, **caractérisé en ce que** l'on produit dans le gaz résiduaire de l'installation de traitement de biogaz un gaz résiduaire énergétiquement valorisé, tandis que l'on abandonne la recirculation du gaz résiduaire de l'installation

5. Procédé selon l'une des revendications 1 à 4, **caractérisé en ce que** l'on mélange le gaz résiduaire de l'installation de traitement de biogaz avec du biogaz brut, du biogaz brut partiellement traité et/ou du biogaz naturel et on le conduit à la chaudière à eau chaude pour une combustion en vue de produire de la chaleur, pour couvrir les besoins en énergie variables de la production de biogaz.
